# EUROPEAN PATENT APPLICATION

(11) **EP 4 783 186 A1**
(43) Date of publication of application: **29.07.2026**
(21) Application number: 25153286.7
(22) Date of filing: 22.01.2025
(51) Int. Cl.: G16H 30/20

(54) **METHOD FOR SENDING MEDICAL IMAGE DATA TO A CLOUD STORAGE SYSTEM, AND METHOD FOR RETRIEVING MEDICAL IMAGE DATA FROM A CLOUD STORAGE SYSTEM**

(71) Applicant: Siemens Healthineers AG, 91301 Forchheim (DE)
(72) Inventor: Anandaram, Ashok Shidlaghatta, 560079 Bangalore (IN); Manuel, Sujith, 91052 Erlangen (DE)
(74) Representative: Siemens Healthineers Patent Attorneys

(57) **Abstract**

Method for sending medical image data to a cloud storage system, in particular for archiving, and method for retrieving medical image data from a cloud storage system

The invention relates to a computer-implemented method for sending medical image data to a cloud storage system, wherein the method comprises the steps:
- obtaining, at a edge system, medical image data and a storage request from a medical system, the storage request requesting storage of the medical image data, wherein the medical system and the edge system are parts of a local network;
- storing the medical image data in an incoming cache of the edge system;
- sending, by the edge system, a storage response to the medical system via the local network;
- sending the medical image data from the incoming cache to a cloud storage system, the cloud storage system being separated from the local network, wherein the uploading to the cloud storage system is decoupled with regard to time from the sending of the storage response.

## Description

Independent of the grammatical term usage, individuals with male, female or other gender identities are included within the term.

The invention relates to computer-implemented methods for sending medical image data to a cloud storage system, in particular for archiving. The invention further relates to computer-implemented methods for retrieving medical image data from a cloud storage system. The invention further relates to computer-implemented methods for storing a medical data set in a storage tier of a cloud storage system.

Medical images are commonly stored and archived in image archival systems, such as PACS systems. Normally, these systems are hosted locally at a hospital site. Thus, the local on-premises IT infrastructure, in particular the long term storage infrastructure, owned and operated by the hospitals is used for storing medical image data. Often, legal requirements are in force, requiring medical service providers to archive all or a substantial portion of the acquired patient image data.

This causes several problems. For smaller medical institutions, such as smaller hospitals or smaller medical practices, the necessary investment in archiving and storage infrastructure may be too high. Also, large medical service providers and/or large medical institutions may face problems, as one medical institution may include several satellite sites, such as satellite hospitals, which are locally spread across different locations. To fulfill the legal archiving requirements, each these sites would have to have dedicated local IT infrastructure. However, often this dedicated IT infrastructure is not available and/or investing in such local IT infrastructure does not pay off in the long run. Thus, a solution is required which enables all of the disconnected satellite sites to archive medical image data.

At some local sites, the local IT infrastructure and local archiving solutions, like PACS, may be built and used. However, these sites, may search for fail-safe storage solutions mechanisms in case of disaster recovery situations. For example, if the local PACS is down due to a hardware failure etc., medical image data should still be accessible without any or with limited delays.

Addionally, in some multi-site setups, e.g., multi-site institutions or a network of collaborating medical institutions, where there are distributed storage systems in place, it may be desired to make information exchange between the storage systems possible.

Thus, the invetion has the objective to solve the above-explained problems. In particular, the invention shall facilitate the storage and archival of medical image data. In particular, the invention shall provide a cost-effective and locally independent solution for storing and/or archiving medical image data. In particular, the invention shall integratable into existing IT-infrastructure of medical institutitons, e.g. for replacing a local PACS system or for extening and/or augmenting the functionalities of a local PACS system.

This task is solved by the features of the independent claims. Further advantageous examples are included in the dependent claims.

According to an aspect of the invention, a computer-implemented method for sending medical image data to a cloud storage system is provided. In particular, the medical image data may be sent to the cloud storage system for archiving. The method comprises a step of obtaining, at an edge system, medical image data and a storage request from a medical system. The storage request requests storage of the medical image data. The medical system and the edge system are parts of a local network. A further step includes storing the medical image data in an incoming cache of the edge system. A further step includes sending, by the edge system, a storage response to the medical system via the local network. The storage response indicates that the storage request for the medical image data is accepted. In particular, the storage response may indicate that the medical image data is successfully stored in the incoming cache. A further step includes sending the medical image data from the incoming cache to a cloud storage system, in particular for archiving, the cloud storage system being separated from the local network. The sending to the cloud storage system is decoupled, in particular with regard to time and/or transferred data size, from the sending of the storage response.

Preferably, the storage request and the storage response comply with the DICOM standard. Preferably, the sending of the medical image data to the cloud storage system does not comply with the DICOM standard.

In general, "Obtaining" may include "collecting" and/or "determining" and/or "receiving" and/or "retrieving" and/or "querying a database to obtain", independently how the data/information/element to be obtained is specifically embodied. In particular, obtaining may include receiving at the edge system, via a DICOM interface, medical image data. In particular, obtaining, at the cloud storage system, may include determining of an appropriate tier identifier.

In particular, the edge system is a part or a component of the local network. In particular, the local network is located at a medical institution, such as a hospital or a doctor's office etc. The local network may be formed by data processing software and/or hardware at said institution. In particular, the local network is a self-contained system. In particular, the local network may be a DICOM network connecting multiple DICOM nodes, wherein a DICOM node may be represented by the edge system and/or by a medical system, e.g.

Examples of the local network may include, but are not limited to, private or public local-area networks (LAN), wireless local-area networks (WLAN), metropolitan-area networks (MAN), wide-area networks (WAN), the Internet, and/or combinations thereof. The communication over the local network may include wired and/or wireless communications according to one or more standards and/or via one or more transport mediums. The communication over the local network may be performed in accordance with various communication protocols such as Transmission Control Protocol and Internet Protocol (TCP/IP), User Datagram Protocol (UDP), and IEEE communication protocols. In one example, the local network may include wireless communications according to Bluetooth specification sets or another standard or proprietary wireless communication protocol. In another example, the local network may also include communications over a cellular network, including, e.g., a GSM (Global System for Mobile Communications), CDMA (Code Division Multiple Access), or EDGE (Enhanced Data for Global Evolution) network.

One or more medical systems may be connected to the local network. In other words: The edge system and the at least one medical system may be part of a common local network. The communication in the local network may comply with the DICOM standard. In particular, some, preferably all, of the components of the local network may include, may be assigned with or may have a DICOM Application Entity Title (AET). By contrast, the components of the cloud storage system, in particular the storage tiers may not have any DICOM Application Entity Titles.

The term "edge system" is to be understood broadly and may refer to any suitable software system or hardware system, adapted for communication with the medical system in the local network and for communication with the cloud storage system. For example, the edge system may be a computing unit, which is adapted to execute the functionalities of an the edge system, as described in context with present method. In particular, the edge system may be located at an institution like for example a hospital or a doctor's office etc. In other words, the edge system may include data processing software and/or hardware at said institution. As explained above, the edge system may be a participant in local communication according to the DICOM standard, wherein the edge system may have a specific DICOM Application Entity Title. In particular the edge system may be considered as entry point from the edge system into the cloud storage system. In particular the edge system may be adapted to translate between the DICOM standard, used for communication at the local network, another communication protocol or standard, used for communication between the edge device and the cloud storage system.

In the present application, the term "medical system" is to be understood broadly and refers to a systems for creating, generating and/or processing medical data, in particular medical image data. Examples of medical systems may be storage systems such as components for image storage and processing, e.g. a picture archiving and communication system (PACS). Further examples may be for managing and reviewing medical reports, such as a reporting system, and/or for radiology reporting and/or review, e.g. radiology information system (RIS). The systems may comprise components or processes, which may be adapted for computerized provider order entry (CPOE), clinical decision support, patient monitoring, population health management (e.g., population health management system (PHMS), health information exchange (HIE), etc.), healthcare data analytics and/or cloud-based image sharing. The systems may include electronic medical record systems (e.g., electronic medical record system (EMR), electronic health record system (EHR), electronic patient record (EPR), personal health record system (PHR), etc.), and/or other health information systems (e.g., clinical information system (CIS), hospital information system (HIS), patient data management system (PDMS), laboratory information system (LIS), cardiovascular information system (CVIS), treatment planning systems (TPS), oncology information systems (OIS), etc.). Further examples may be workstations of a medical IT infrastructure and/or imaging modalities for generating and/or processing medical image data.

The medical image data may be or may include a two-dimensional image. The medical image data may be or may include a three-dimensional image. The medical image may be or may include a four-dimensional image, where there are three spatial and one time-like dimensions. The medical image data may comprise a plurality of individual medical images.

The medical image data comprises image data, for example, in the form of a two- or three-dimensional array of pixels or voxels. Such arrays of pixels or voxels may be representative of color, intensity, absorption or other parameters as a function of two or three-dimensional position, and may, for example, be obtained by suitable processing of measurement signals obtained by a medical imaging modality or image scanning facility.

A medical image may be a radiology image depicting a body part of a patient. Accordingly, it may contain two or three-dimensional images of the patient's body part. The medical image may be representative of an image volume or a cross-section through the image volume. The patient's body part may be comprised in the image volume.

A medical imaging modality corresponds to a system used to generate or produce medical image data. For example, a medical imaging modality may be a computed tomography system (CT system), a magnetic resonance system (MR system), an angiography (or C-arm X-ray) system, a positron-emission tomography system (PET system) or the like. Specifically, computed tomography is a widely used imaging method and makes use of "hard" X-rays produced and detected by a spatially rotating instrument. The resulting attenuation data (also referred to as raw data) is processed by a computed analytic software producing detailed images of the internal structure of the patient's body parts. The produced sets of images are called CT-scans which may constitute multiple series of sequential images to present the internal anatomical structures in cross sections perpendicular to the axis of the human body. Magnetic Resonance Imaging (MRI), to provide another example, is an advanced medical imaging technique which makes use of the effect magnetic field impacts on movements of protons. In MRI machines, the detectors are antennas and the signals are analyzed by a computer creating detailed images of the internal structures in any section of the human body.

Accordingly, the depicted body part of the patient in general will comprise a plurality of anatomies and/or organs. Taking a chest image as an example, the medical image may show lung tissue, the rib cage, lymph nodes and others.

The medical image data may comprise a plurality of images or image slices. The slices respectively show a cross-sectional view of the image volume. The slices may comprise a two-dimensional array of pixels or voxels as image data. The arrangement of slices in the medical image data may be determined by the imaging modality or by any post-processing scheme used. Further, slices may artificially be defined in the imaging volume spanned by the medical image data. Optionally, this may happen as a function of the image data comprised in the medical image data in order to optimally pre-process the medical image data for the ensuing diagnostic workflow.

The medical image data may be a two-dimensional pathology image data set, i.e., a so-called whole-slide image. A whole-slide image may show a tissue slice or slide of a patient. In particular, the image data may include at least one mammography image. Mammography is an X-ray imaging method used to examine the breast, e.g., for the early detection of cancer and other breast diseases. It serves both as a diagnostic and screening tool. During a mammogram, the breast is compressed between two firm surfaces to spread out the breast tissue, and then an X-ray captures black-and-white mammography.

In the present disclosure, the medical image data may be present in a standard image format, such as the Digital Imaging and Communications in Medicine (DICOM) format. The apart from medical images per se, the medical image data may also include metadata and/or structured reports, e.g. In particular, the term medical image data may be used as a synonym for data in a standard image format, independently whether the data actually includes images or other data, such as structured reports or metadata. The image data may be stored in a memory or computer storage system such as a Picture Archiving and Communication System (PACS), a Radiology Information System (RIS), and the like. Whenever DICOM is mentioned herein, it shall be understood that this refers to the "Digital Imaging and Communications in Medicine" (DICOM) standard, for example according to the DICOM PS3.1 2020c standard (or any later or earlier version of said standard).

The medical system may transfer medical image data to the edge system for storage. For this, a storage request may be sent to the edge system. The storage request may be accompanied by the medical image data to be stored. The edge system may comprise an interface to the local network, for information exchange, e.g., for receiving storage requests and/or medical image data to be stored and/or for sending storage responses. A storage response may indicate acceptance, success or failure of the storage request. The storage response may be generated independently of the sending process or the uploading process. In particular, the storage response may be generated based on success or failure of storing the medical image data in the incoming cache.

In particular, the requesting and responding between the medical system and the edge system may be executed according to the DICOM Storage Service. The DICOM Storage service may be used to transfer DICOM images and other related digital data from a DICOM node to another DICOM node. Thus, by means of the DICOM Storage service medical data may be exchanged among multiple devices over the local network.

According to the DICOM Storage service a DICOM Storage SCP, i.e. Service Class Provider, awaits incoming connections. The incoming connections may established by DICOM Storage SCUs, i.e., Service Class Users. Thus, a DICOM Storage SCU, in order to send medical image data, such as DICOM images (or other related data), to another node acting as a DICOM Storage SCP, actively connects to that SCP node. When the connection is established, the DICOM SCU may send the storage request in combination with the actual data to transfer. After that, the SCU may wait for a storage response by the SCP, the storage response specifying whether the storage process was successful and/or whether the storage request is accepted.

In particular, the SCU may send a C-STORE-RQ (request) message to the SCP. The request may include or may be combined with the actual dataset to transfer. The SCU expects the SCP to return a C-STORE-RSP (response) message, communicating success or failure of the storage request.

In an exemplary embodiment, the edge system may be operated as DICOM Storage SCP. The medical systems of the local network may be operated as DICOM Storage SCUs. In particular, the storage request may be a C-STORE-RQ (request) message. The storage response may be a C-STORE-RSP (response) message. If medical image data is provided from the edge system to one or more medical systems, the edge system may be operated as DICOM Storage SCU and the medical systems of the local network may be operated as DICOM Storage SCPs.

Thus, the interaction between the edge system and the medical systems in the local network may be similar to the interaction with a PACS system. In particular, the edge system may comprise the same interfaces and may be adapted to provide the same services to the nodes in the local network as a PACS system. Preferably, a PACS system of an institution may be replaced by an edge system and a cloud system executing the claimed method, without any changes to the IT-infrastructure of the institution. Alternatively, an edge system and a cloud system executing the claimed methods may be used for augmenting the functionalities of a PACS system of an institution, without any changes to the IT-infrastructure of the institution.

The incoming cache and/or the below-mentioned outgoing cache may be a storage device. In particular, the cache may be a hardware or software component that temporarily stores data, in particular medical image data, to make future data retrieval and/or sending faster and more efficient. Normally, the storage space of a cache is used by devices, browsers, and applications to quickly access frequently used information without needing to reload it from a main storage every time the use is required. In the present disclosure, the cache is used as an intermediate storge or buffer for the received medical image data. Thus, the sending of the medical image data to the cloud system is decoupled from the receiving of the medical data and the confirming of the storage request at the edge system. In particular, the edge system may confirm a successful storage of the medical image data, once the data is stored in the incoming cache, and not, once the data is uploaded or sent to the cloud storage system.

After sending of the medical image data to the cloud storage system, the data may be deleted from the patient cache. This allows for an efficient use of local storage resources. The medical data may be stored at the patient cache for a certain period of time. The time period may be pre-defined and/or the time period may be flexibly adjusted based on the intended use of the medical image data. For example, there may be situations, where a re-use of the medical image data in the near future may be likely, e.g. when a follow-up exam is scheduled. In this case, it may be advantageous to keep a copy of the medical image data locally in the edge system to allow for a fast retrieval of the image data in a follow-up situation.

Storing the medical image data in the incoming cache further allows pre-processing of the medical image data before the medical image data is sent to the cloud system, as further explained below.

In particular, the cloud storage system is separated from the edge system. The cloud storage system may hosted by a server which is not placed at the institution which hosts the edge system. The cloud storage system may be adapted for archiving data, in particular medical data. The edge system and the cloud storage system may be spatially separated. In addition or as an alternative, the cloud system may have no direct access to data which is stored or processed in the edge system. The cloud storage system may only access such data with special permission and/or if the data is actively sent to the cloud storage system. Thus, the cloud storage system and the edge system may be considered as independent information silos. Preferably, when exchanging medical data, such as medical image data, between the edge system and the cloud storage system, data protection guidelines are observed. In particular, the cloud storage system is not part of the self-contained edge system, and vice versa.

The cloud storage system and the edge system may be at least temporarily connected to each other for data/information transfer and/or exchange. The systems may be connected by a common information exchange connection. The information exchange connection may be realized using a network connection. In an example, the network may be realized as local area network (LAN), the internet, or a private network, such as an intranet or a wide area network (WAN).

As explained, due to the intermediate storage of the medical image data in the incoming cache, the sending of medical data to the cloud storage system is decoupled, in particular with regard to time and/or upload speed, from the sending of the storage response. In particular, the storage response may be sent based on the storing of the medical image data in the incoming cache, and not based on a successful sending of the storage data to the cloud storage system. The storage response may indicate, if the storage of the medical data in the incoming cache was successful or not. In particular, the storage response may be sent before the medical image data is completely sent to the cloud.

Thus, with the method according to the invention, medical image data may be stored/ archived externally in a cloud storage system, while the operating behavior for the medical systems in a local network does not change in comparison to the use of an on-site PACS system. However, the storage size of the cloud storage system can be flexibly adjusted, the cloud storage system may include a fail-safe mechanism. In particular, the cloud storage system is independent from any local distribution of individual medical sites, as it can be interacted with from any location by means of an edge system.

Thus, the invention provides a cost-effective and locally independent solution for storing and/or archiving medical image data, as it is integratable into existing IT-infrastructure of medical institutitons without requiring changes to the local IT-infrastrucute. E.g., the invention may be used for replacing a local PACS system or for extening and/or augmenting the functionalities of a local PACS system.

Preferably, communication in the local network, in particular the storage request and the storage response exchanges between the edge system and the medical system, is based on the DICOM standard. Preferably, the sending of the medical image data from the incoming cache to the cloud storage system is not based on the DICOM standard. Thus, the medical systems in the local network may interact with the edge system according to the DICOM standard. Although the communcation between the edge system and the cloud storage system may not comply with the DICOM standard, the medical systems in the local network may communicate via the edge system with the cloud storage as if the cloud storage system was part of the local network and as if the cloud storage system had a DICOM AET.

Preferably, the method further comprises the step:
- minimizing the medical image data, in particular patient identifiers, included in the medical image data, while the medical image data is stored in the incoming cache.

The minimizing of patient identifiers included in the medical image data may be executed before the medical image data is sent to the cloud storage system. Preferably, minimizing may include anonymizing and/or pseudonymizing the medical image data.

In the present disclosure, pseudonymization refers to a procedure in which person-related data is replaced by unique identifiers, allowing to trace data back to its origins, and to preserve a patient-to-data mapping. In anonymization, all person-related data that could allow backtracking is nullified. Therefore, anonymized data may not contain any hint on the corresponding patient, which may be required due to legal privacy requirements, e.g.

Preferably, the medical image data is pseudonymized before the medical image data is sent to the cloud storage system. As a result, privacy of the medical image data and of sensitive patient information is ensured. However, using a pseudonymized identifier associated with a specific patient for pseudonymizing the medical image data, still allows for re-identifying of the specific patient. For this, a patient-identifier-mapping may be stored in a local database at the edge system. Once the medical image data is retrieved from the cloud storage system to the local edge system, the local database can be queried to retrieve the patient identifier corresponding to the pseudonymized identifier and the medical image data can be re-identified, to allow for further processing by the medical systems in the local network.

Due to the possibility of safely storing and archiving patient data in a cloud storage, storage burdens on local hospital storage, such as a PACS, can be significantly relieved. The privacy of patient data is not impaired due to the pseudonymization.

According to a preferred embodiment, the method further comprises the step of slicing the medical image data, in particular, while the medical image data is stored in the incoming cache. In particular, slicing may include dividing the medical image data into smaller, in particular more manageable, subsets. The slicing may be based on characteristics of an information exchange connection between the edge system and the cloud storage system and/or based on characteristics of the medical image data.

In particular, the slicing may include slicing the medical image data into a plurality of subsets. In particular, each subset may have a smaller storage size than the medical image data itself. In particular, the slicing may be based on available bandwidth, available cache size and/or requirements of cloud storage system, in particular the storage tier, such as restrictions of the file-size to be stored in a storage tier of the cloud storage system.

As the medical image data is stored in the incoming cache to decouple the sending process from the processes in the local network, the sending process and/or the slicing of the medical image data may be conducted in a manner taking into account the peculiarities of the information exchange connection between the cloud storage system and the edge system. For example, an available bandwidth for sending the medical image data may be taken into account. If a high bandwidth is available for sending the medical image data to the cloud storage system, the medical image data may be divided into bigger slices than when only a small bandwidth is available for sending the medical image data. Preferably, for the slicing, the number of parallel connections between the edge system and the cloud storage system may be taken into account.

In a preferred embodiment, the medical image data may be grouped into data packages before being sent to the cloud. In other words, the medical image data may be aggregated in the incoming cache and an aggregated bundle of data may be transferred to the cloud. This approach may improve the structure of the medical image data and may also allow for an efficient transfer of medical image data between the edge system and the cloud storage system.

Preferably, a sending speed or an upload speed of the medical image data is adjusted based on an available bandwidth between the edge system and the cloud storage system. This allows for a faster and more reliable transfer of the medical image data. In particular, the sending speed of sending the medical image data to the cloud may be different from the sending speed with which the medical image data is received from the medical systems.

Preferably, the cloud storage system may include multiple storage tiers. This may allow for a hierarchical storage management. The storage tiers may include high-cost storage tiers and low-cost storage tiers. Each storage tier may be tailored towards a specific use case and therefore may include different characteristics. In particular, the storage tiers may include different access times, a different access/storage structure, a different level of changeability of data, a different level of convertibility of data, a different level of data security, a different level of black-out security, a different frequency of backups and/or a different data format. In a preferred embodiment, the cloud storage system includes at least a hot tier, wherein the hot tier allows a real-time or immediate retrieval of the stored medical image data. The hot tier may be a highspeed storage device such as a solid-state drive or an array of solid state drives. In addition or as an alternative, the cloud storage system may include an archive tier, wherein the archive tier allows an long-term storage, in particular archiving, of the medical image data. The archive tier may include a slower access time than the hot tier. The archive tier may be a hard disk drive or an array of hard disk drives, e.g. A tier, in particular the archive tier may be distributed over multiple local sites. This makes the tier robust against black-out and/or fire. In other words, due to the underlying mechanisms data stored in the hot tier may be retrieved basically without a delay. Data stored in the archive tier may be retrieved with a certain delay.

The archive tier may provide an immutable storage of the medical image data. The medical image data may be stored in the archive tier for a period of multiple years, e.g., two years or five years, or even tens of years or thirty years, e.g. The length of storage of the medical image data may depend on the legal requirements in place at a certain location.

In particular, the cloud storage system may include at least one hot tier. The hot tier may allow a quick/immediate access and/or retrieval of the stored medical image data. Within the framework of this disclosure, immediate access means that the image data may be retrieved from the hot tier to the edge system, basically just in time or in real-time, in particular within milliseconds, seconds or minutes. The immediate accessibility may be caused by a specific data storage architecture of the hot tier and/or by other suitable measures.

The hot tier and/or the archive tier may include a storage versioning, such that any change to the medical image data is version controlled. This allows to protect the medical image data, e.g., against ransomware attacks. For each version of the medical image data, immutability may be enabled. This may be especially relevant for the archive tier, as the data stored in the archive tier is normally not changed any more. The data immutability may be enabled during a specified and/or configurable time-period, e.g. for 30 years. Thus, accidental deletions of medical image data can be avoided.

Moving data between the hot tier and the archive tier may be triggered and/or controlled by the edge system in the local network. For this, the method may include at least one of the following steps:
- determining, based on the medical image data, a tier identifier, the tier identifier identifying one of multiple storage tiers of the cloud storage system, in which the medical image data shall be stored;
- sending the tier identifier to the cloud storage system.

The tier identifier may be received by the cloud storage system, and the corresponding medical image data may be stored in the corresponding tier, as further detailed below.

Alternatively, the medical image data may be moved between the hot tier and the archive tier based on rules and/or algorithms stored and/or executed in the cloud storage environment.

The selection of the storage tier and/or the corresponding determination of a tier identifier may be based on the medical image data and/or other medical data which is available for the patient. For example, a patient history and/or an examination schedule and/or a treatment schedule of the patient may be taken into account. According to an example, the medical image data may be stored in the hot tier, when it is likely that the medical image data may be re-used soon, e.g., when a follow-up treatment or follow-up exam is scheduled. For example, a worklist or work schedule of an evaluator of the medical image data may be taken into account. In particular, the medical image data, which is schedule for evaluation in a certain future time period, may be sent to, moved to or stored in the hot tier, such that it can be immediately accessed by the evaluator, when it is required.

The selection of the storage tier and/or the corresponding determination of a tier identifier may be based on time. E.g., the medical data may be stored in the hot tier for a certain period of time, e.g. for a certain number of days or months, e.g., 6 months, and may be moved to the archive tier after the period of time, if it has not been used during the storage period in the hot tier. For example, a timestamp of the medical image data may be taken into account for calculating a time for moving the image data from one tier to another. The timestamp may be or may include the time of last access to the data. The timestamp may be or may include the time of creation of the data.

The selection of the storage tier and/or the corresponding determination of a tier identifier may be based on usage patterns of users of the medical systems. For example, the usage of medical image data may be tracked. Rules for moving the image data between the storage tiers may be derived from the tracked usage.

The above-explained approaches for the selection of the storage tier and/or the corresponding determination of a tier identifier may be used in isolation or may be combined. This internal automatic storage tiering allows for an efficient use of the hot tier storage space, which is usually more cost intensive to install and maintain than the storage space in the archive tier. Thus, intelligently moving the medical image data between storage tiers, makes the invention a cost-effective solution.

In a preferred embodiment, the method further comprises the steps:
- determining, based on data available in the local network, an updated tier identifier, the updated tier identifier identifying one of multiple storage tiers of the cloud storage system, to which medical image data, which is stored in the cloud storage system shall be shifted;
- sending the updated tier identifier to the cloud storage system.

In consequence, based on the updated tier identifier, medical image data stored in the cloud storage system may be shifted from one storage tier to another storage tier. For example, image data may be shifted from the hot tier to the archive tier, or vice versa. The updated tier identifier may be determined based on data available in the local network. In particular, the updated tier identifier may be determined based on a prediction of future use of the medical image data. The updated tier identifier may be determined based on work lists or working schedules, e.g., schedules for the annotation or evaluation of medical images. Other examples of working lists or working schedules, which may be taken into account may be schedules for follow-up scans, pathology working lists or tumor-boards. Based on the schedules, it may be derived, when the medical image data may be required to be available in the local network.

According to an example, the communication within the medical system in the local network may be monitored by the edge system. For example, an FHIR-stream or similar data streams may be monitored. Once it is detected based on the communication in the medical system, that medical image data may be required to be retrieved from the cloud storage system, e.g., because a patient is loaded into a worklist, the corresponding image data may be shifted to the hot tier, in order to be available for retrieval. Similarly, data may be shifted or stored in the hot tier, once a scheduled data for an examination or evaluation comes closer. This may be reflected by selecting a corresponding tier identifier and/or by determining a corresponding updated tier identifier.

In principle, the explanations provided above with regard to the selection of a storage tier, also apply for determining an updated tier identifier. In this framework, the criteria for selecting a storage tier for first time storage may correspond to the criteria for determining an updated tier identifier.

According to an example, the medical image data may be stored in the hot tier or may be shifted to the hot tier, when it is likely that the medical image data may be re-used soon, e.g., when a follow-up treatment or follow-up exam is scheduled. As local data for determining the corresponding tier update, for example, a worklist or work schedule of an evaluator of the medical image data may be taken into account. In particular, the medical image data, which is scheduled for evaluation in a certain future time period, may be sent to, moved to or stored in the hot tier, such that it is available for fast download.

In a preferred embodiment, the method may comprise at least one of the steps:
- obtaining, from a medical system in the local network, a storage commitment request, in particular DICOM storage commitment request, and
- transmitting, to the cloud storage system, a cloud storage commitment request based on the storage commitment request;
- obtaining, from the cloud storage system, a cloud storage commitment report, based on the cloud storage commitment request;
- transmitting, to the medical system, a storage commitment report, in particular DICOM storage commitment report, based on the cloud storage commitment report.

Preferably, the storage commitment request and the storage commitment report comply with the DICOM standard. Preferably, the cloud storage commitment request and the cloud storage commitment report do not comply with the DICOM standard. Thus, at the edge device, the received DICOM storage commitment request may be converted to a non-DICOM cloud storage commitment request, which may be send to the cloud storage system. On the other hand, at the edge device, the received non-DICOM cloud storage commitment report may be converted to a DICOM storage commitment report and may be sent to the medical system. The edge device may be seen a translator between the DICOM communication in the local network and the non-DICOM communication to and from the cloud storage system.

The above-explained commitment request and report, in particular DICOM storage commitment request and report, may be interpreted as kind of re-assurance service. In this sense, the medical system may request confirmation from the edge system that the data is securely and safely stored. As the medical image data is forwarded by the edge system to the cloud storage system, also the DICOM storage commitment request may be forwarded or translated, such that it may be forwarded to the cloud storage system in order to reliably determine, whether the data has been successfully stored in the cloud storage system. The received response, i.e. the cloud storage commitment report from the cloud storage system may be forwarded to the medical system in the local network. Alternatively, the received response may be translated into a DICOM storage commitment report, which may be sent to the medical system. Thus, the invention allows for providing commitment reports via a standard interface, in particular a DICOM standard interface. There is no need to enable the medical systems in the local network to communicate directly with the cloud storage system. As there is an internal, preferably non-DICOM, communication between the edge system and the cloud storage system, the medical systems can interact with the edge system in the standard way, preferably according to the DICOM standard, as it was a local storage and archival system.

The provision of storage commitment reports may be implemented as DICOM Storage Commitment service. Similar to the above explained example of DICOM Storage service, a medical system in the local network may be operated as SCU. The edge system may be operated as SCP. When a SCU issues a DICOM Storage request to a SCP, as explained above, in order to transfer DICOM data, the Storage request may be accepted by the SCP by returning a successful C-STORE-RSP message. The corresponding response message of SCP may indicate that the transferred DICOM data is accepted. However, there is no guarantee the transferred DICOM data has successfully been stored by the SCP.

Therefore, the SCU may require an additional confirmation that the medical data is securely stored. DICOM Storage Commitment service allows a DICOM client application, e.g., a medical system to request a commitment from a server, e.g., from the edge system, for safekeeping of certain DICOM images and related data.

In the framework of the DICOM Storage Commitment service, a SCU may send a DICOM commitment request to a SCP to commit to storage and safekeeping of medical image data, in particular of a specific set of DICOM images and/or related DICOM data. The commitment request may be sent via a N-ACTION-RQ message. The SCP may accept the SCU's request by returning a N-ACTION-RSP message. The SCP will then asynchronously notify the client, i.e. the SCU, about its commitment to store and safekeep the requested set of DICOM objects. For this, a DICOM commitment report may be sent to the SCU. The commitment report may be framed as a DICOM N-EVENT-RPT message. Preferably, the report will only indicate a successful commitment, if the SCP commits to safekeep all the DICOM objects requested by the SCU in its previous N-ACTION-RQ message. If the SCP cannot commit to safely store one or more of the requested DICOM instances, then the report may include a failure notification.

In one scenario, a medical system, in particular a DICOM modality, may acquire medical image data, in particular a set of DICOM studies. The medical image data may be stored in an internal storage of the medical system. Further, the medical images may be forwarded to the edge system for longer-term archiving. For this, the DICOM Storage service may be used, for example. Later, it may be desired to delete the medical image data from the internal storage. However, before deleting the data, a Storage Commitment request may be sent to the edge system, for making sure that a copy of the data is available in the cloud storage system. The storage commitment request may cover the entire medical image data, or some of the medical image data, which has been selected for deletion.

The edge system may interact with the cloud storage system to determine whether the image data, for which approval of commitment is request, has successfully been stored in the cloud storage system. For this a cloud storage commitment request may be sent to the cloud storage system. The answer from the cloud storage system may be a cloud storage commitment report, in which the cloud storage system approves or declines that the medical image data is successfully stored in the cloud storage system. The communication between the edge system and the cloud storage system may be according to the DICOM Storage Commitment service, or may be an alternative communication.

Based on the report from the cloud storage system, the edge system may generate a storage commitment report for sending it to the medical system. In one embodiment, the edge system may forward the cloud storage commitment report as storage commitment report to the medical system. Alternatively or in addition, the edge system may generate the storage commitment report based on the cloud storage commitment report.

If the edge system answers by sending a successful Storage Commitment report, then it can be derived that the medical image data which shall be deleted from the internal storage of the medical system is safely stored in the cloud storage system. Hence, it is safe to delete the medical image data from the internal storage of the medical system.

The cloud storage system may include or may be related to a storage commitment database, in which information about successfully stored medical image data is bundled. When it shall be determined whether a certain image data has been stored successfully, the storage commitment database may be queried based on the storage commitment request of the edge system, to retrieve information whether the corresponding image data has been stored successfully. Based on this information, the cloud storage commitment report may be generated. Hence it is not necessary to search the actual archiving storage of the cloud storage system but the information about successfully stored medical image data is easily accessible in the database.

The cloud storage system may include or may be related to a series completion worker, which automatically adds an entry to the commitment database, once the corresponding image data is fully uploaded/sent to the cloud storage system and/or is successfully stored in the cloud storage system.

According to an aspect, a computer implemented method for retrieving medical image data from a cloud system and/or for providing medical image data to a medical system is disclosed. The method comprises a step of obtaining, at an edge system, an image query from a medical system. The image query identifies medical image data to be provided by the edge system. The medical system and the edge system are parts of a local network. A step includes retrieving/downloading, by the edge system, the identified medical image data from a cloud storage system. The cloud storage system is separated from the local network. A step includes storing the retrieved medical image data in an outgoing cache of the edge system. A step includes providing, by the edge system, the medical image data from the outgoing cache to the medical system. The retrieving from the cloud storage system is decoupled, in particular with regard to time and/or speed, from the providing of the medical image data to the medical system.

This part of the invention focuses on providing and/or retrieving medical image data, which has been stored and/or archived in the cloud storage system, to medical systems in the local network. The explanations provided in the context with the method for sending medical image data to the cloud storage system, are also valid in the context with the method for retrieving/providing medical image data. To keep the present disclosure concise, the explanations are not repeated. In particular, it is emphasized that also in the case of providing and/or retrieving medical image data, which has been stored and/or archived in the cloud storage system, the communication in the local network may be according to the DICOM standard, whereas the communication between the edge system may not comply with the DICOM standard.

Preferably, the image query and/or the providing of the medical image data to the medical system comply with the DICOM standard. Preferably, the retrieving of the medical image data from the cloud storage system does not comply with the DICOM standard.

In the image query, a medical system may identify medical image data, which shall be loaded from the cloud storage system. For example, this may be the case, if the medical image data may be required for a follow-up examination, and/or the medical image data shall be sent to another institution.

The feature "identifying of the medical image data" shall be understood broadly within the framework of this application. In particular, the image query shall include any information, based on which medical image data may be identified. The information may be a unique DICOM key and/or DICOM tag, a patient identifier, an exam identifier, a collection of search parameters, and/or any other suitable information.

The edge system may determine, whether the requested image data is available at the cloud storage system, and may retrieve the identified medical data from the cloud storage system. For the retrieving/downloading, similar features may be applied as for the above-described sending of medical data. E.g., a download bandwidth available for information exchange between the cloud storage system and the edge system may be taken into account, when a download speed is determined and set.

The retrieved medical data may be stored in an outgoing cache. The outgoing cache may be designed similarly to the incoming cache. The outgoing cache may be separated from the incoming cache, or the caches may share the same resources. Due to storing the data in the outgoing cache, the retrieving of the data is decoupled, in particular with regard to time and/or data size, from providing the data to the medical system. In other words, the data is buffered in the outgoing cache. As said, the explanations made in the context of the incoming cache are in principle also applicable to the outgoing cache.

The retrieved medical data may be pre-processed in the outgoing cache. In particular, pseudonymized data may be re-identified. For this, a pseudonymized identifier included in the medical image data may be replaced by a non-pseudonymized identifier. The non-pseudonymized identifier may be retrieve from a patient database based on the pseudonymized identifier. In particular, the medical image data may be sliced into smaller data packages. The size of the data packages may be based on an available bandwidth for transmitting the medical image data from the edge system to the requesting medical system. Also, other pre-processing steps, which have been explained in combination with the incoming cache may be applied. In some embodiments, the pre-processing methods applied by the incoming cache may be applied inversely at the outgoing cache. For example, if data is aggregated at the incoming cache into a data-bundle for sending the data to the cloud, the data bundle may be sliced in the outgoing cache after retrieving it, in order to prepare it for transmission to the medical system.

From the outgoing cache, the medical image data may be provided to the medical system. For example, the medical image data may be sent to the requesting medical system.

Due to the intermediate storage in the outgoing cache, the retrieving from the cloud storage system is independent from the providing of the medical image data to the medical system. In particular, the transmission speed of the retrieving may be different from the transmission speed of the providing of the medical image data. This may be due to the fact, that the available bandwidth for the transmission may be different and/or due to the fact, that medical system may only be capable of receiving a certain amount of data in a particular time frame.

For example, the retrieving from the cloud storage system may be conducted with a higher transmission speed than the transmission to the requesting medical system, as normally, the medical systems are not capable of handling as many incoming data packages as the edge system or as the cloud storage system.

According to examples, the retrieving from the cloud storage system may at least partly overlap with providing the medical image data to the medical systems. This means that the transmission of the medical image data to the medical systems may already be started, even when not the full medical image data is retrieved from the cloud storage system in order to avoid unnecessary delays in the provision of the medical image data to the medical systems.

In particular, the edge system may be capable of monitoring a transmission connection to the medical system with regard to successful transmissions of data packets. If it is detected, that a number of failed transmission is above a certain threshold, the transmission speed may be reduced to give the medical system more time to process the medical image data received from the edge system. In particular, the edge system may be capable of managing associations, in particular a number and/or type of associations, between the edge system and the medical systems, based on responses from the medical systems. In particular, a current association parameter may be adjusted.

In particular, the providing of the medical image data to the medical systems may be executed based on the DICOM C-MOVE service and/or protocol. The medical system may send a DICOM C-MOVE-RQ message to the edge system. The edge system may answer the C-MOVE-RQ message with a C-MOVE-RSP message.

For actually providing the medical images to the medical system, the edge system may use the DICOM C-STORE service, similarly, as explained above.

Thus, in a preferred embodiment, the edge system may be operated as a DICOM Storage SCP. The edge system may be operated as a DICOM storage commitment SCP. The edge system may be operated as a DICOM query retrieve SCP. The edge system may be operated as a DICOM Storage SCU when providing the requested medical image data to the medical system. In particular, the edge system may provide one, some or all of the described functionalities.

As said, preferably, the communication between the edge system and the medical systems complies with the DICOM standard. This is not a necessary requirement for the connection between the edge system and the cloud storage system. The connection between the edge system and the cloud storage system may be optimized with regard to other goals, for example with regard to transmission speed and/or security.

Preferably, the method further comprises the steps:
- determining, based on the image query, a tier identifier, the tier identifier identifying one of multiple storage tiers of the cloud storage system, in which the medical image data is stored;
- retrieving the medical image data from the determined tier of the cloud storage system.

As explained above, the cloud storage system may include multiple tiers with different characteristics. By intelligently determining the tier, at which the medical data is available, the communication between the cloud storage system and the edge system may be facilitated. In particular, the edge system may directly send a retrieval/download request to the appropriate tier or may refer to the appropriate tier in a retrieval/download request.

In a preferred embodiment, the method may include a step of obtaining, at the edge system, an image find request from a medical system, the image find request identifying medical image data to be found by the edge system. A step may include determining, by the edge system, whether the image data is available in the cloud storage system. A step may include providing by the edge system, a response message to the medical system, indicating whether the image data is available at the cloud storage system.

Preferably, the image find request and the image find response comply with the DICOM standard. Preferably, the determining, whether the image data is available in the cloud storage system does not comply with the DICOM standard.

Preferably, the communication between the medical system and the edge system with regard to the image request may be structured according to the DICOM C-Find Service and/or the DICOM C-FIND protocol. The C-FIND service is an operation by which relevant patient information may be queried and provided. The medical system may send a DICOM C-FIND-RQ message to the edge system, specifying at least one certain key or tag, for which the processing shall search. The edge system may respond with a C-FIND-RSP message, specifying whether the image data relating to the received keys or tags is available.

Preferably, the edge system may be adapted to pre-emptively retrieve medical image data to the outgoing cache before an image query is received.

In one scenario, pre-emptive retrieval of medical image data may be conducted upon receiving a find request and responding to the find request, the edge system may pre-emptively retrieve the corresponding medical image data from the cloud storage system. Hence, once an image query from the medical image system is received, the data is already available in the outgoing cache of the edge system, and can be provided to the medical system without delay.

Similarly, pre-emptive retrieval of medical image data may be conducted based on a treatment schedule, an examination schedule of the patient, a worklist including medical image data to be viewed at a medical system, e.g., a radiologist's or evaluator's worklist, etc.

Preferably, explained aspects of the managing of the communication and transmission of medical image data between the medical systems and the edge system and/or between the edge system and the cloud storage system is conducted by proxies. Thus, the incoming cache may be connected to the cloud storage system via an upload proxy or upload worker. In addition or as an alternative, the outgoing cache may be connected to the cloud storage system via a download proxy or a download worker.

Preferably, the edge system comprises a DICOM standard interface to the local network. Hence, the medical systems can interact with the edge system according to the DICOM standard. This facilitates integration of the edge system in the local network. It enables upgrading the functions of existing medical IT local networks with cloud storage capabilities without the need for changing the existing IT infrastructure of the local network.

According to an aspect, a computer-implemented method for storing medical image data in a storage tier of a cloud storage system is provided. The cloud storage system may be embodied as described in the context of the above-described methods. The method comprises a step including obtaining, at a cloud storage system, medical image data from an edge system in a local network. The cloud storage system comprises a plurality of storage tiers and is separated from the local network. A step includes obtaining, at the cloud storage system, a tier identifier, the tier identifier identifying the storage tier, in which the medical image data shall be stored. A step includes selecting a selected storage tier of the plurality of storage tiers based on the tier identifier. A step includes storing the medical image data in the selected storage tier of the cloud storage system.

Preferably, the cloud storage system may include a storage redundancy service. The medical data may be copied automatically, e.g. after a pre-defined time period or when a certain action is conducted, such as the transfer from the hot tier to the archive tier, to a redundancy repository of the cloud storage system. Hence, the medical data is protected from transient hardware failures, network or power outages, and natural disasters. In particular, this enables the cloud storage system to serve as a back-up module and as an archiving module. If an outage renders a part of the cloud storage system unavailable, then a failover can be initiated to the redundancy repository to rapidly restore the medical data.

Preferably, the cloud storage system may include a study database, in which information is provided, in particular DICOM tags and/or DICOM keys, about the medical image data, which is stored in the cloud storage system. Thus, when a medical system requests finding certain medical image data in the cloud storage system, it can be avoided to search the entire storage, in particular the archiving tier, of the cloud storage system. Instead, the information about the stored medical image data is available in the study database. In particular it may be available which medical image data is stored in the cloud storage device, and optionally, in which storage tier the medical image data is currently stored.

Preferably, the medical data may be evaluated in the cloud storage system. For example, medical image data may be transmitted to the cloud storage system for evaluation and analysis of the medical image data, e.g., for determining medical findings based on the image data. For this, the medical image data may be stored in the hot tier of the cloud storage system, such that it can be changed easily.

A medical finding may relate to corresponding medical image in the medical image data. A medical finding may indicate a certain condition or pathology of the patient. The condition or pathology may be relevant for the diagnosis of the patient.

In other words, the medical finding may relate to an anatomical structure that differentiates the patient from other patients. Medical findings may be located within different organs of the patient (e.g., within the lung of a patient, or within the liver of a patient) or in between the organs of the patient. In particular, a medical finding may also relate to a foreign body.

In particular, a medical finding may relate to a neoplasm (also denoted as "tumor"), in particular, a benign neoplasm, an in-situ neoplasm, a malignant neoplasm and/or a neoplasm of uncertain/unknown behavior. In particular, a medical finding may relate to a nodule, in particular, a lung nodule. In particular, a medical finding may relate to a lesion, in particular, a lung lesion.

Medical findings may be classified according to their type or category. This type or category is called "finding type". A finding type may specify the general nature of medical finding. Further, the finding type may specify the anatomy or organ in which a medical finding has been found. According to some implementations, the finding type may also be conceived as a label of the medical finding. For instance, finding types may be lung nodule, liver nodule, cyst, rib fracture, undefined lesion, etc.

In some cases, the medical finding may be that there is no finding in the medical image data.

A medical finding may be determined by a human evaluator of the medical image data and/or by a computer aided detection algorithm, which may generally be configured to detect candidate medical findings in medical images. For instance, the findings detection algorithms may have two stages: the detection stage for detecting potentially relevant patterns in image data and the classification stage for classifying the potentially relevant patterns either as candidate medical findings or as false positives to be discarded. In principle, a plethora of functionalities and methods is known for such computer aided detection and classification of candidate medical findings - all of which may be implemented in the findings detection algorithms. According to examples, the findings detected by an algorithm may be reviewed by an human evaluator.

The medical findings may be included in the medical image data, such that they are available, when the image data is retrieved from the cloud storage system to the outgoing cache of the edge system. Hence, one use of the invention may be within a teleradiology framework.

According to a further aspect, a data processing apparatus comprising means for carrying out the steps of the method according to any of the claimed embodiments is provided.

According to a further aspect, a computer program product is provided, wherein the computer program product comprises instructions, which, when the program is executed by a computer, cause the computer to carry out any of the above-mentioned embodiments.

The computer program product, such as a computer program means, may be embodied as a memory card, USB stick, CD-ROM, DVD or as a file which may be downloaded from a server in a network. For example, such a file may be provided by transferring the file comprising the computer program product from a wireless communication network.

According to a further aspect, a computer-readable storage medium is provided, comprising instructions, which, when executed by a computer, cause the computer to carry out the steps of the method according to any of the above-mentioned embodiments.

The invention is also related to a system especially configured and/or adapted for running and/or executing the computer implemented method according to the invention. The system may comprise a processing unit with data processing apparatus, adapted to execute the method according to any of the above-described embodiments. Further, the processing unit may include storage means, capable of providing the functionality of the incoming cache and/or of the outgoing cache.

In the following, the solution according to the invention is described with respect to the claimed edge system and cloud storage system as well as with respect to the claimed methods.

Features, advantages or alternative embodiments herein can be assigned to the other claimed objects and vice versa. In other words, claims for the edge system or the cloud storage system can be improved with features described or claimed in the context of the methods. In this case, the functional features of the method are embodied by objective units of the edge system or the cloud storage system.

All in all, the claimed methods and systems facilitate the long-term storage and archival of medical image data. The claimed methods and systems may be integrated and/or executed within existing hardware structures without requiring changes to the existing hardware structure, as the communicate to the medical systems of a local network via standard interfaces, such as DICOM interfaces, which would be provided by a PACS system.

Further possible implementations or alternative solutions of the invention also encompass combinations - that are not explicitly mentioned herein - of features described above or below with regard to the embodiments. The person skilled in the art may also add individual or isolated aspects and features to the most basic form of the invention.

Further embodiments, features and advantages of the present invention will become apparent from the subsequent description and dependent claims, taken in conjunction with the accompanying drawings, in which:
- Fig. 1: shows a schematical illustration of an embodiment of a healthcare IT infrastructure;
- Fig. 2: shows a schematical illustration of a method for sending medical image data to a cloud storage system for archiving; and
- Fig. 3: shows a schematical illustration a method for retrieving medical image data from a cloud storage system.

In the Figures, like reference numerals designate like or functionally equivalent elements, unless otherwise indicated. To improve the understandability of the present specification some of the described examples are only explained in the context of generating a query message. However, the teachings can be likewise applied to the generation of a response message.

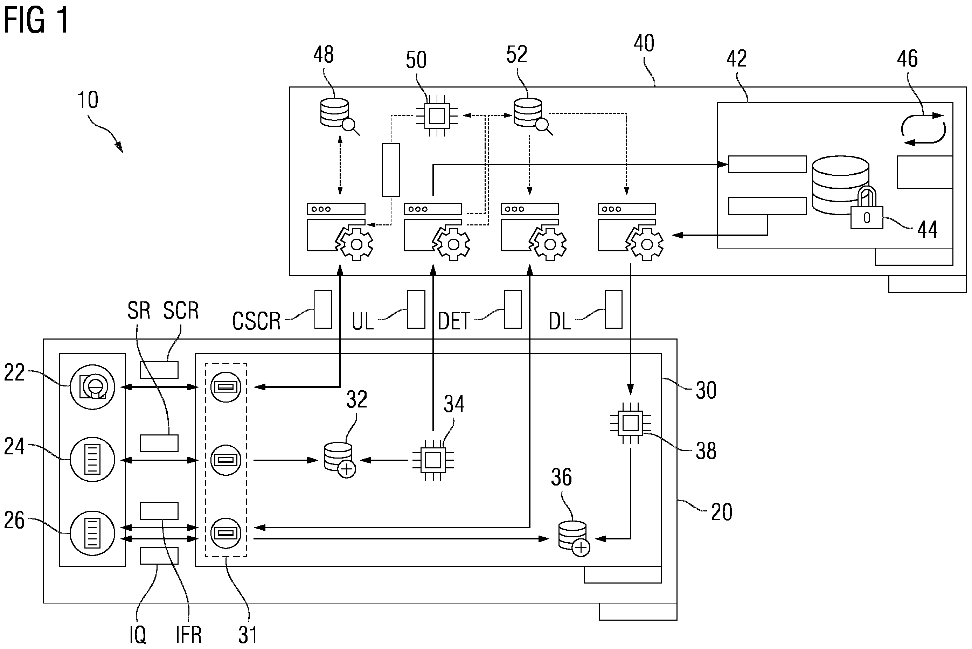

Figure 1 shows a schematical illustration of an embodiment of a healthcare IT infrastructure 10. The healthcare infrastructure includes a local network 20 and cloud platform 40, which is separated from the local network 20. In particular, the local network 20 may be spatially and/or functionally distanced from the cloud platform 40.

In an example, the local network 20 may represent a hospital IT-infrastructure. The local network 20 may be an institutional network connecting multiple medical systems 22, 24, 26 at a site of a medical institution. For example, the local network 20 may include medical systems, such as imaging modalities 22, PACS 24 and/or at least one, preferably a plurality of workstations 26.

The imaging modality 22 may be adapted to acquiring medical image data, e.g. from a patient. The PACS 24 may be adapted to store, archive and/or organize medical image data. The workstations 26 may fulfill a plurality of different functions. In particular, workstations 26 may be used for viewing medical image data, for analyzing, annotating and/or evaluating medical image data, for communicating medical image data, etc.

The local network 20 further includes an edge system 30, the edge system 30 serves as a transmitter between the medical systems 20 and the cloud platform 40 with the cloud storage system 42. In other words, the edge system 30 provides an interface 31 to the medical systems, i.e. to the imaging modality 22, to the PACS 24 and/or to the workstation 26.

Preferably, the local network 20 is a DICOM network. Preferably, the interface 31 for interacting with the edge system 30 is designed according to the DICOM standard, such that the edge system 30 can be easily integrated into the local network 20, preferably without changing the structure of the local network 20. Due to the standard interface, the medical systems may not notice any change when communicating with the edge system 30 in comparison to communicating with a standard local system for organizing and/or archiving medical image data, such as a PACS.

In the present embodiment, the local network 20 includes a PACS 24 as well as an edge system 30. However, in other embodiments, a PACS 24 may be at least partially replaced by an edge system 30, such that the local network 20 may not include a local PACS system anymore or such that the required local storage of a local PACS system is reduced. Instead, the combination of edge system 30 and cloud storage system 42 may at least partially replace or augment the functionalities of a local PACS system 24.

The medical systems 22, 24, 26 may communicate to the edge system 30 over the interface 31. In particular the medical systems 22, 24, 26 may exchange storage request/responses SR, storage commitment requests/reports SCR, image find requests/responses IFR, image queries IQ and corresponding responses, etc., with the edge system 30.

A storage request SR.1 may be used by the medical systems 22, 24, 26 to request storage of medical image data from the edge system 30. The edge system 30 may issue a storage response SR.2, if the medical image data is accepted for storage and/or if the medical image data is successfully stored in an incoming cache 32 of the edge system 30.

The medical systems 22, 24, 26 may send a storage commitment request SCR.1 to the edge system 30, when an approval is requested that medical data has successfully been stored, in particular in the cloud storage system 42. To approve or decline the request, the edge system 30 may issue a storage commitment report SCR.2 and send it to the medical systems 22, 24, 26.

An image find request IFR.1 may be sent to the edge system 30, if a medical system 22, 24, 26 wants to determine, whether specific medical image data is available for retrieval from the cloud storage system 42. The edge system 30 may respond with an image find response IFR.2.

For requesting the provision of medical image data, the medical systems 22, 24, 26 may send an image query IQ. As a response, the edge system 30 may provide the medical image data to the requesting medical system 22, 24, 26.

Incoming image data received by the edge system 30 from a medical system 22, 24, 26 may be stored in the incoming cache 32, in order to make the sending of the medical image data to the cloud storage system 42 independent from the receiving of the medical images from the medical systems 22, 24, 26. The sending process may be controlled by an upload worker 34 serving as a proxy for controlling and managing the sending/upload process of the medical image data.

If image data is retrieved from the cloud storage system 42, the image data may be buffered in the outgoing image cache 36, such that the retrieving process is independent of the process of actually providing the medical image data to the medical systems 22, 24, 26. The edge system 30 may include a download worker serving as a proxy for intelligently controlling the download process of medical image data and/or the providing of medical image data to the medical systems 22, 24, 26.

The edge system 30 is in information exchange connection with the cloud computing platform 40, in particular with the cloud storage system 42 of the cloud computing platform 40.

The cloud storage system 42 includes at least two storage tiers. In the present embodiment, the cloud storage system 42 includes an archive tier 44 for long time storage and a hot tier 46 for short-time storage and/or fast access to the stored medical image data. In particular, the medical image data may be moved or transferred between the storage tiers of the cloud storage system 42 and/or copied between tiers of the storage system based on characteristics of the imaging data, such as content of the image data, intended use of the image data, time of acquisition of the image data, time of last change to the image data, etc.

For example, the image data may be available at the hot tier 46 for immediate retrieval for a certain time period. After that time period, the data may be copied or shifted to the archive tier 44. Once the data is shifted to the archive tier, it may be deleted from the hot tier 46, independently of the specific shifting criteria. Changes to the archived image data may be version controlled, wherein each version of the image data may be immutable to prevent accidental deletions or changes to the data.

The cloud computing platform 40 and/or the cloud storage system 42 may further include a storage commitment database 48. The storage commitment database 48 may include information required for answering cloud storage commitment requests CSCR.1. In particular, it may include information which medical image data has been successfully stored in the cloud storage system 42. Hence, when responding to a cloud storage commitment request CSCR.1, it can be avoided to search the entire cloud storage system 42 for corresponding medical data. Instead, the storage commitment database 48 can be efficiently searched and a cloud storage commitment report CSCR.2 can be generated based on the result extracted from the storage commitment database 48.

The storage commitment database 48 may be populated by means of a series completion worker 50, which generates an entry in the storage commitment database 48, once the corresponding medical image data has been successfully stored in the cloud storage system 42.

For easier access and to facilitate the overview over the stored medical files, the cloud computing platform 40 may further include a study database 52. The study database 52 may include a storage-reduced version of the medical image data stored in the cloud storage system 42, e.g., a summary of the metadata of the medical data stored in the cloud storage system 42. Thus, the study database 52 may be used for efficiently getting in overview over the medical data stored in the cloud storage system 42, e.g., if a query for medical image data is received and if it needs to be determined whether the medical image data is available in the cloud storage system 42.

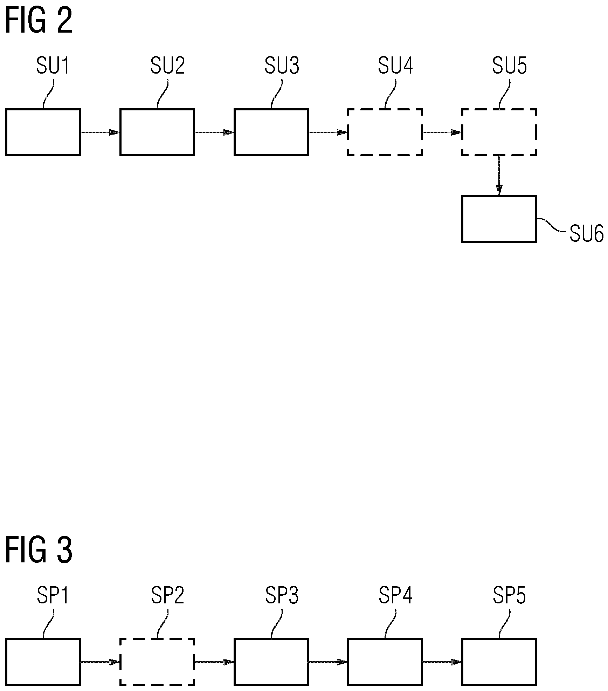

Figure 2 shows a schematical illustration of a method for sending or uploading medical image data to a cloud storage system 42. Some or all of the steps of the method for uploading medical image data may be executed before or in combination with the method for providing medical image data to a medical system 22, 24, 26.

In a step SU1, medical image data and a storage request from a medical system 22, 24, 26 is obtained at an edge system 30. By means of the storage request SR.1, the medical system 22, 24, 26 requests storage of the medical image data. As explained above, the medical system 22, 24, 26 and the edge system 30 are parts of a local network 20.

In a step SU2, the obtained medical image data is stored in an incoming cache 31 of the edge system 30. Preferably, directly after storing the medical image data in the incoming cache 31, in a step SU3, a storage response SR.2 is sent to the medical system 22, 24, 26 via the local network 20. The storage response SR.2 may indicate that the medical image data is successfully received by the edge system 30, that the storage request SR.1 is accepted and/or that the storage of the medical image data is successful. In particular, the storage response SR.2 may be sent, before the uploading of the medical image data to the cloud storage system 42 is initiated. In particular, the storage response SR.2 may be sent, before the uploading of the medical image data to the cloud storage system 42 is finished.

Thus, the medical systems 22, 24, 26 receive a direct feedback from the edge system 30, indicating that the storage request is accepted. Preferably, the feedback is not dependent on the actual uploading process, which is managed in the background. The interaction between the local network and the edge system 30 is similar to a PACS, in particular, as the edge system 30 provides standard interfaces, such as DICOM, to the local network.

Optionally, in a step SU4, pre-processing of the medical data may be conducted, before the medical data is uploaded to the cloud storage system 42. In particular, the medical data may be minimized and/or the medical data may be bundled or divided into appropriate data packages to facilitate and/or accelerate the upload of the medical data to the cloud storage system.

Optionally, in a step SU5, a tier identifier may be determined, the tier identifier identifying a storage tier of the cloud storage system 42 to which the medical data shall be uploaded. In particular, it may be determined at the edge system 30, whether the medical image data shall be uploaded to the hot tier or to the archive tier of the cloud storage system 42.

The determination of the storage tier may be based on the characteristics of the medical image data. For example, it may be determined in an automated process, whether the medical image data includes medical findings, requiring the medical image data to be viewed in a follow-up examination. For this, the medical image data may include a specific attribute in the metadata. In addition or as an alternative, an algorithm may be provided, the algorithm being adapted or trained to process an examination and/or treatment plan of a patient, to identify, whether the medical image data will be re-loaded within a certain time frame to be viewed again.

In a step SU6, the medical image data is uploaded from the incoming cache 32 to a cloud storage system 42. In particular, if a target storage tier for the medical image data is identified, the medical image data may be uploaded to the target storage tier of the cloud storage system 42.

As explained, the cloud storage system 42 is separated from the local network 20. Due to the storing of the medical image data in the incoming cache 32, the uploading to the cloud storage system is decoupled from the sending of the storage response SR.2. In particular, the storage response SR.2 may be sent before the uploading is finished, or, in some cases, the storage response SR.2 may be sent before the uploading has even started.

This allows, dynamically adjusting the upload speed of the medical image data. In particular, the upload speed of the medical image data to the cloud storage system 42 may be independent of the speed with which the medical image data is received by at the edge system 30 from the medical systems 22, 24, 26.

Figure 3 shows a schematical illustration a method for providing medical image data to a medical system. Some or all of the steps of the method for providing medical image data to a medical system 22, 24, 26 may be executed after or in combination with the method for uploading medical image data.

In a step SP1, an image query IQ from a medical system 22, 24, 26 is obtained at an edge system 30. In the image query IQ medical image data to be provided by the edge system 30 is specified. As explained, the medical system 22, 24, 26 and the edge system 30 are parts of a common local network 20.

Optionally, in a step SP2, a tier identifier is determined based on the image query IQ, the tier identifier identifying one of multiple storage tiers of a cloud storage system 42, in which the medical image data is stored. As explained above, the cloud storage system is separated from the local network. This step may be done at the edge system 30, in particular, if the cloud storage system 42 includes multiple separate storage instances, which have to be addressed independently from each other. Alternatively, or in addition, this step may be conducted at the cloud storage system 42 or the cloud computing platform 40, in particular, if the medical image data is distributed within the cloud storage system 42.

In a step SP3, the edge system 30 may download or retrieve the identified medical image data from the cloud storage system 42. In a step SP4, the downloaded medical image data is stored in an outgoing cache 36 of the edge system 30.

In a step SP5, the medical image data is provided from the outgoing cache 36 to the medical system 22, 24, 26. Due to the storage of the medical image data in the outgoing cache 36, the downloading from the cloud storage system 42 is independent from the providing of the medical image data to the medical system 22, 24, 26. In particular, the medical image data may be provided independent with regard to time. This means that the starting point for providing the medical image data to the medical systems 22, 24, 26 may be independent from the starting point or finishing point of downloading and storing the medical image data. In particular, the medical image data may be first entirely downloaded to the outgoing cache 36, and afterwards distributed to the medical system 22, 24, 26. Alternatively, or in addition, the speed of downloading the medical image data from the cloud storage system 42 may be different from the speed of providing the medical image data to the medical systems 22, 24, 26. Hence, the outgoing cache 36 fulfills a kind of buffering function.

The method for uploading medical image data to a cloud storage system 42 and/or the method for providing medical image data to a medical system 22, 24, 26 may be executed may be executed by an edge system 30, e.g., as described in context with Figure 1. Where applicable, features or explanations provided in context with the method for providing medical image data may be also implemented in the context of the method for uploading medical image data, and vice versa.

All in all, the presented systems and methods facilitate the transmission and storage of medical image data between local institutions/networks and cloud storage systems. A efficient way for storing medical images in a cloud is provided, which may be offered as a service to local institutions, e.g., to upgrade the available storage size, to provide a fail-safe-archiving solution and/or to replace local PACS systems entirely. The storage size available at the cloud storage system for a local institution may be dynamically adjusted, based on the need of the local institution. Hence, available cloud storage can be used more efficiently. Via the edge system, the medical systems in the local network can interact with the cloud storage system as if it was part of the local network. In particular, the edge system may include a standard interface to the local network, such as a DICOM interface.

The various illustrative logical blocks, modules, circuits, and algorithm steps described in connection with the embodiments disclosed herein may be implemented as electronic hardware, computer software, or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans may implement the described functionality in varying ways for each particular application, but such implementation decisions should not be interpreted as causing a departure from the scope of this disclosure or the claims. Embodiments implemented in computer software may be implemented in software, firmware, middleware, microcode, hardware description languages, or any combination thereof. A code segment or machine-executable instructions may represent a procedure, a function, a subprogram, a program, a routine, a subroutine, a module, a software package, a class, or any combination of instructions, data structures, or program statements. A code segment may be coupled to another code segment or a hardware circuit by passing and/or receiving information, data, arguments, parameters, or memory contents. Information, arguments, parameters, data, etc., may be passed, forwarded, or transmitted via any suitable means including memory sharing, message passing, token passing, network transmission, etc.

The actual software code or specialized control hardware used to implement these systems and methods is not limiting of the claimed features or this disclosure. Thus, the operation and behavior of the systems and methods were described without reference to the specific software code being understood that software and control hardware can be designed to implement the systems and methods based on the description herein.

When implemented in software, the functions may be stored as one or more instructions or code on a non-transitory computer-readable or processor-readable storage medium. The steps of a method or algorithm disclosed herein may be embodied in a processor-executable software module, which may reside on a computer-readable or processor-readable storage medium. A non-transitory computer-readable or processor-readable media includes both computer storage media and tangible storage media that facilitate transfer of a computer program from one place to another. A non-transitory processor-readable storage media may be any available media that may be accessed by a computer. By way of example, and not limitation, such non-transitory processor-readable media may comprise RAM, ROM, EEPROM, CD-ROM or other optical disk storage, magnetic disk storage or other magnetic storage devices, or any other tangible storage medium that may be used to store desired program code in the form of instructions or data structures and that may be accessed by a computer or processor. Disk and disc, as used herein, include compact disc (CD), laser disc, optical disc, digital versatile disc (DVD), floppy disk, and Blu-ray disc where disks usually reproduce data magnetically, while discs reproduce data optically with lasers. Combinations of the above should also be included within the scope of computer-readable media. Additionally, the operations of a method or algorithm may reside as one or any combination or set of codes and/or instructions on a non-transitory processor-readable medium and/or computer-readable medium, which may be incorporated into a computer program product.

The preceding description of the disclosed embodiments is provided to enable any person skilled in the art to make or use the embodiments described herein and variations thereof. Various modifications to these embodiments will be readily apparent to those skilled in the art, and the principles defined herein may be applied to other embodiments without departing from the spirit or scope of the subject matter disclosed herein. Thus, the present disclosure is not intended to be limited to the embodiments shown herein but is to be accorded the widest scope consistent with the following claims and the principles and novel features disclosed herein.

While various aspects and embodiments have been disclosed, other aspects and embodiments are contemplated. The various aspects and embodiments disclosed are for purposes of illustration and are not intended to be limiting, with the true scope and spirit being indicated by the following claims.

## Claims

1. Computer-implemented method for sending medical image data to a cloud storage system (42), in particular for archiving, wherein the method comprises the steps:
- obtaining, at an edge system (30), medical image data and a storage request from a medical system (22, 24, 26), the storage request requesting storage of the medical image data, wherein the medical system (22, 24, 26) and the edge system (30) are parts of a local network (20);
- storing the medical image data in an incoming cache (32) of the edge system (30);
- sending, by the edge system (30), a storage response to the medical system (22, 24, 26) via the local network (20), the storage response indicating that the storage request for the medical image data is accepted;
- sending the medical image data from the incoming cache (32) to a cloud storage system (42), in particular for archiving, the cloud storage system (42) being separated from the local network (20), wherein the sending of the medical image data to the cloud storage system (42) is decoupled from the sending of the storage response
wherein, preferably, the storage request and the storage response comply with the DICOM standard, and, preferably, the sending of the medical image data to the cloud storage system does not comply with the DICOM standard.

2. Method according to the preceding claim, wherein the method further comprises the step:
- anonymizing or pseudonymizing the medical image data while the medical image data is stored in the incoming cache (32).

3. Method according to any one of the preceding claims, wherein the method further comprises the step:
- slicing the medical image data while the medical image data is stored in the incoming cache (32), wherein the slicing is based on characteristics of an information exchange connection between the edge system (30) and the cloud storage system (42).

4. Method according to any one of the preceding claims, wherein an upload speed for sending the medical image data to the cloud storage system (42) is adjusted based on an available bandwidth between the edge system (30) and the cloud storage system (42).

5. Method according to any one of the preceding claims, wherein the method further comprises the steps:
- determining, based on the medical image data, a tier identifier, the tier identifier identifying one of multiple storage tiers of the cloud storage system (42), in which the medical image data shall be stored;
- sending the tier identifier to the cloud storage system (42).

6. Method according to any one of the preceding claims, wherein the method further comprises the steps:
- determining, based on data available in the local network, an updated tier identifier, the updated tier identifier identifying one of multiple storage tiers of the cloud storage system (42), to which medical image data, which is stored in the cloud storage system (42), shall be shifted;
- sending the updated tier identifier to the cloud storage system (42).

7. Method according to any one of the preceding claims, wherein the method further comprises the steps:
- obtaining, from a medical system (22, 24, 26), a storage commitment request, and
- transmitting, to the cloud storage system (42), a cloud storage commitment request based on the storage commitment request;
- obtaining, from the cloud storage system (42), a cloud storage commitment report, based on the cloud storage commitment request;
- transmitting, to the medical system (22, 24, 26), a storage commitment report based on the cloud storage commitment report,
wherein, preferably, the storage commitment request and the storage commitment report comply with the DICOM standard, and, preferably, the cloud storage commitment request and the cloud storage commitment report do not comply with the DICOM standard.

8. Computer-implemented method for retrieving medical image data from a cloud system, wherein the method comprises the steps:
- obtaining, at an edge system (30), an image query from a medical system (22, 24, 26), the image query identifying medical image data to be provided by the edge system (30), wherein the medical system (22, 24, 26) and the edge system (30) are parts of a local network (20);
- retrieving, by the edge system (30), the identified medical image data from a cloud storage system (42), the cloud storage system (42) being separated from the local network (20);
- storing the retrieved medical image data in an outgoing cache (36) of the edge system (30);
- providing, by the edge system (30), the medical image data from the outgoing cache (36) to the medical system (22, 24, 26), wherein the retrieving of the medical image data from the cloud storage system (42) is decoupled from the providing of the medical image data to the medical system (22, 24, 26),
wherein, preferably, the image query and/or the providing of the medical image data to the medical system comply with the DICOM standard, and, preferably, the retrieving of the medical image data from the cloud storage system does not comply with the DICOM standard.

9. Method according to claim 8, wherein the method further includes:
- obtaining, at the edge system (30), an image find request from a medical system (22, 24, 26), the image find request identifying medical image data to be found by the edge system (30),
- determining, by the edge system (30), whether the image data is available in the cloud storage system (42),
- providing by the edge system (30), an image find response to the medical system (22, 24, 26), indicating whether the image data is available at the cloud storage system (42),
wherein, preferably, the image find request and the image find response comply with the DICOM standard and, preferably, the determining, whether the image data is available in the cloud storage system (42) does not comply with the DICOM standard.

10. Method according to any one of the preceding claims, wherein the edge system (30) comprises a DICOM standard interface to the local network (20), wherein, preferably, the communication in the local network complies with the DICOM standard and, preferably, the communication between the edge system and the cloud storage system does not comply with the DICOM standard.

11. Computer-implemented method for storing medical image data in a storage tier of a cloud storage system (42), wherein the method comprises the steps:
- obtaining, at a cloud storage system (42), medical image data from an edge system (30) in a local network (20), the cloud storage system (42) comprising a plurality of storage tiers and being separated from the local network (20);
- obtaining, at the cloud storage system (42), a tier identifier, the tier identifier identifying the storage tier, in which the medical image data shall be stored;
- selecting a selected storage tier of the plurality of storage tiers based on the tier identifier;
- storing the medical image data in the selected storage tier of the cloud storage system (42).

12. Method according to any one of the preceding claims, wherein the cloud storage system (42) includes at least a hot tier (46) and an archive tier (44), wherein the hot tier (46) allows a real-time retrieval of the stored medical image data and the archive tier (44) allows a long-term storage of the medical image data.

13. A data processing apparatus comprising means for carrying out the steps of the method of any of claims 1 to 12.

14. A computer program product comprising instructions, which, when the program is executed by a computer, cause the computer to carry out the steps of the method of any of claims 1 to 12.

15. A computer-readable storage medium comprising instructions, which, when executed by a computer, cause the computer to carry out the steps of the method of any of claims 1 to 12.
